# EUROPEAN PATENT SPECIFICATION

(11) **EP 4 365 168 B1**
(45) Date of publication and mention of the grant of the patent: **15.10.2025**
(21) Application number: 22845449.2
(22) Date of filing: 22.07.2022
(51) Int. Cl.: C07C 235/06, A61K 31/08, A61K 9/127, A61K 47/18

(54) **POLYOL-MODIFIED LIPID COMPOUND AND PREPARATION METHOD AND APPLICATION THEREOF**
POLYOLMODIFIZIERTE LIPIDVERBINDUNG UND HERSTELLUNGSVERFAHREN UND ANWENDUNG DAVON
COMPOSÉ LIPIDIQUE MODIFIÉ PAR UN POLYOL ET PROCÉDÉ DE PRÉPARATION ET APPLICATION CORRESPONDANTS

(30) Priority: 23.07.2021 CN 202110839337
(43) Date of publication of application: 08.05.2024
(73) Proprietor: Jenkem Technology Co., Ltd. (Tianjin), Tianjin 300462 (CN)
(72) Inventor: WANG, Qingbin, Tianjin 300462 (CN); HU, Shuzhen, Tianjin 300462 (CN); HAO, Jing, Tianjin 300462 (CN); ZHANG, Baitao, Tianjin 300462 (CN); ZHU, Changyou, Tianjin 300462 (CN); GUO, Jun, Tianjin 300462 (CN); ZHAO, Xuan, Tianjin 300462 (CN)
(74) Representative: HGF
(86) International application number: PCT/CN2022/107381
(87) International publication number: WO 2023/001286

(56) References cited:
- EP-B1- 2 663 548
- WO-A1-2018/081480
- WO-A1-2019/089828
- WO-A1-2021/030701
- CN-A- 1 882 693
- CN-A- 106 795 096
- CN-A- 110 167 922
- CN-A- 110 352 071
- CN-A- 113 402 405
- CN-A- 114 276 535
- JP-A- H09 309 869
- US-A1- 2015 203 446

## Description

### TECHNICAL FIELD

The present invention relates to the technical field of medicines, and in particular to a polyglycol-modified lipid compound, and a preparation method therefor and use thereof.

### BACKGROUND

Gene therapy refers to the use of molecular biological methods to introduce a target gene into a patient's body and express it to correct or compensate for diseases caused by gene defects and abnormalities, and also refers to the introduction of nucleic acids into cells to inhibit the expression of a target gene (gene silencing) or increase the expression of a target gene (gene activation) to achieve the purpose of treating diseases. With the progress of biotechnology in recent years, gene therapy drugs have achieved rapid development, bringing huge development prospects to the field of biological drugs. American Alnylam company developed a series of gene drugs by adopting RNAi technology and successfully marketed four RNAi drugs: Onpattro (patisiran), Givlaari (givosiran), Oxlumo (lumasiran) and Leavio (inclisiran), wherein Leavio (inclisiran) is a biological drug for treating hyperlipidemia, and the successful marketing of the drug widens the field of gene therapy from the traditional field of treating rare genetic diseases to the field of treating common diseases, which brings new opportunities for RNAi drugs.

Nucleic acid drugs, due to their intrinsic properties such as electronegativity, susceptibility to degradation by nucleases, cannot efficiently penetrate cell membranes into cells and are rapidly degraded *in vivo,* so a suitable delivery system is required to stably deliver nucleic acids to a target site and take effect. A common difficulty in the development of mRNA and siRNA is how to deliver them efficiently into cells at the target site. During the delivery process, it is also necessary to consider how to avoid rapid clearance, avoid degradation by nucleases, and improve escape after endocytosis, and the like.

At present, the delivery systems for nucleic acids mostly use different types of liposomes, e.g., lipid nanoparticles (LNPs), GalNac, lipopolyplexes (LPPs), for delivery. LNPs can be generally prepared from four types of lipids in a certain proportion, the four types of lipids typically including a cationic lipid, a neutral lipid, a steroid lipid, and a polymer conjugated lipid, wherein the polymer conjugated lipid refers to a polyethylene glycol (PEG) lipid.

Some polyethylene glycol lipids are reported in the prior art. For example, patent document EP2663548B1 discloses in example 9 that a pegylated lipid corresponding to formula (I) wherein X=CH, L2=L1=NHC(O), L3=C(O)X(h=0, j=2 or C(O)NH h=0; j=3; Rb=H, Y=Me.

CN106795096A, WO2019/089828A1 and WO2018/081480A1 disclose a pegylated lipid corresponding to formula (I) wherein L3=C(O)CH2; L1= L2=direct bond, Y=OMe, X=N.

The compounds are used in formulations for delivering active agents, such as siRNA and microRNA. Patent documents CN110352071A and CN1882693A disclose a polyethylene glycol lipid and use of lipid nanoparticles prepared from the polyethylene glycol lipid in the delivery of a bioactive substance into a body. However, polyethylene glycol itself, as a polymer, is a mixture, which easily causes differences between batches, and adversely affects the quality and stability for drug efficacy of the drug formed therefrom.

### SUMMARY

In order to overcome the defects of the prior art, the present invention provides a compound, and a preparation method therefor and use thereof.

More specifically, the compound may be selected from the following structures: 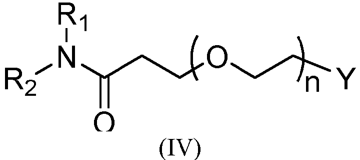 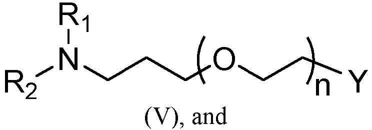 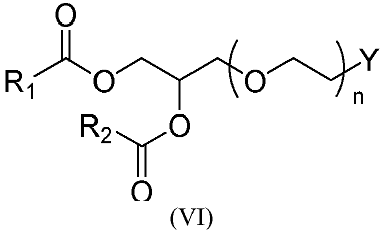 wherein R1 and R2 are each independently hydrocarbyl containing 6 to 30 carbon atoms; n is an integer of 30 to 90; Y is a terminal group.

In some embodiments of the present invention, the compound has the following structure:

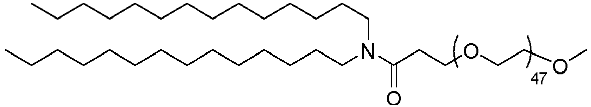

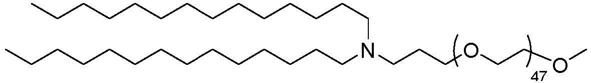

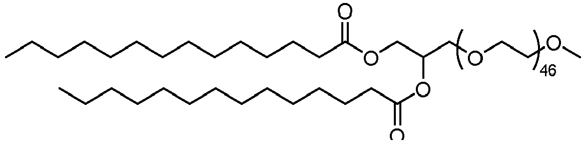

In a second aspect, the present invention provides a pharmaceutically acceptable salt, an ester, an isomer, a prodrug and a solvate of the compound described above.

In a third aspect, the present invention provides a method for preparing the compound according to the first aspect, which may comprise a step of using 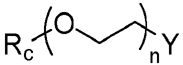 for reaction, wherein n and Y have the corresponding definitions as described in the first aspect of the present invention, and R_{c} is a reactive group, such as, hydroxyl, carboxyl, amino, sulfydryl, maleimide group, succinimide group, alkynyl, azido, aldehyde group, or epoxy.

In a fourth aspect, the present invention provides a lipid composition comprising the compound according to the first aspect, or a pharmaceutically acceptable salt, an ester, an isomer, a prodrug and a solvate thereof. Specifically, the lipid composition may further comprise a cationic lipid, such as one or more of stearamide (SA), lauryltrimethylammonium bromide, hexadecyltrimethylammonium bromide, myristyltrimethylammonium bromide, dimethyldioctadecylammonium bromide (DDAB), 3β-[*N*-(*N*',*N*'-dimethylaminoethane)-carbamoyl]cholesterol (DC-cholesterol), 1,2-ditetradecanoyl-3-trimethylammonium-propane (DMTAP), 1,2-dioctadecyl-3-trimethylammonium-propane (DOTAP) and DOTAP derivatives such as 1,2-di-(9Z-octadecenoyl)-3-trimethylammonium-propane and 1,2-dihexadecanoyl-3-trimethylammonium-propane, 1,2-di-(9Z-octadecenoyl)-3-dimethylammonium-propane (DODAP) and DODAP derivatives such as 1,2-ditetradecanoyl-3-dimethylammonium-propane, 1,2-dihexadecanoyl-3-dimethylammonium-propane and 1,2-dioctadecyl-3-dimethylammonium-propane, 1,2-di-O-octadecenyl-3-trimethylammonium propane (DOTMA), 1,2-dioleoyl-c-(4'-trimethylammonium)-butyryl-sn-glycerol (DOTB), dioctadecylamidoalanyl spermine, SAINT-2, a polycationic lipid 2,3-dioleoyloxy-N-[2(spermine-carboxamido)ethyl]-N,N-dimethyl-1-propanaminium trifluoroacetate (DOSPA), and ((4-hydroxybutyl)azadialkyl)bis(hexane-6,1-diyl)bis(2-hexyldecanoate) (ALC-0315), particularly ALC-0315.

Specifically, the molar ratio of the compound according to the first aspect or the pharmaceutically acceptable salt, the ester, the isomer, the prodrug and the solvate thereof to the cationic lipid in the lipid composition may be 1:0.01-0.1 (e.g., 1:0.01, 1:0.02, 1:0.025, 1:0.03, 1:0.035, 1:0.04, 1:0.045, 1:0.05, 1:0.06, 1:0.08, or 1:0.1), particularly 1:0.03-0.05.

Specifically, the lipid composition may further comprise a neutral lipid, such as one or more of 1,2-distearoyl-sn-glycero-3-phosphocholine (DSPC), 1,2-dipalmitoyl-sn-glycero-3-phosphocholine (DPPC), 1,2-dioleoyl-sn-glycero-3-phosphoethanolamine (DOPE), 1,2-dipalmitoyl-sn-glycero-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoyl phosphatidylcholine (POPC), and 1-palmitoyl-2-oleoyl phosphatidylethanolamine (POPE), particularly DSPC.

Specifically, the molar ratio of the compound according to the first aspect or the pharmaceutically acceptable salt, the ester, the isomer, the prodrug and the solvate thereof to the neutral lipid in the lipid composition may be 1:0.1-0.5 (e.g., 1:0.1, 1:0.15, 1:0.2, 1:0.25, 1:0.3, 1:0.35, 1:0.4, or 1:0.5), particularly 1:0.1-0.3.

Specifically, the lipid composition may further comprise a steroidal lipid, such as avenasterol, β-sitosterol, brassicasterol, ergocalciferol, campesterol, cholestanol, cholesterol, coprosterol, dehydrocholesterol, desmosterol, dihydroergocalciferol, dihydrocholesterol, dihydroergosterol, campesterol, epicholesterol, ergosterol, fucosterol, hexahydrosterol, hydroxycholesterol, lanosterol, photosterol, fucasterol, sitostanol, sitosterol, stigmastanol, stigmasterol, cholic acid, glycocholic acid, taurocholic acid, deoxycholic acid, and lithocholic acid, particularly cholesterol.

Specifically, the molar ratio of the compound according to the first aspect or the pharmaceutically acceptable salt, the ester, the isomer, the prodrug and the solvate thereof to the steroidal lipid in the lipid composition may be 1:0.5-1.5 (1:0.5, 1:0.6, 1:0.7, 1:0.8, 1:0.9, 1:1, 1:1.1, 1:1.2, 1:1.4, or 1:1.5), particularly 1:0.8-1.2.

More specifically, the lipid composition comprises the compound according to the first aspect, and a cationic lipid, a neutral lipid and a steroidal lipid; in some embodiments of the present invention, the composition comprises the compound described in the first aspect, and ALC-0315, DSPC and cholesterol.

Specifically, the lipid composition may further comprise one or more pharmaceutically acceptable excipients, such as carriers, adjuvants, and diluents.

Disclosed herein is a pharmaceutical composition comprising a bioactive substance, and the compound according to the first aspect or the lipid composition according to the third aspect.

Specifically, the bioactive substance may be a small molecule compound, a nucleic acid, a peptide, a protein, or the like

Specifically, the bioactive substance is a nucleic acid, such as DNA or RNA, wherein the DNA may be noncoding DNA (antisense DNA) or coding DNA, and the RNA may be selected from: one or more of an antisense RNA, an saRNA, an mRNA, a lncRNA, an miRNA, an siRNA, a piRNA, a gRNA, a tsRNA, etc., particularly an mRNA or an siRNA

In one embodiment of the present invention, the bioactive substance is an siRNA that inhibits the expression of a target gene, such as an anti-KDR siRNA.

Specifically, the bioactive substance may be used for the prevention and/or treatment of one or more diseases selected from: cancer, inflammation, fibrotic disease, autoimmune disease, disease caused by infection of a pathogen, psychiatric disorder, hematological disorder, chromosomal disease, genetic disease, connective tissue disease, digestive disease, ear-nose-throat disease, endocrine disease, ocular disease, reproductive disease, heart disease, kidney disease, lung disease, metabolic disorder, oral disease, musculoskeletal disease, nutritional disease, skin disease, etc.

Specifically, the molar ratio of the bioactive substance to the compound according to the first aspect or the lipid composition according to the third aspect in the pharmaceutical composition is 0.01-0.5:1 (e.g., 0.01:1, 0.02:1, 0.03:1, 0.04:1, 0.05:1, 0.06:1, 0.07:1, 0.08:1, 0.09:1, 0.1:1, 0.2:1, 0.3:1, 0.4:1, or 0.5:1), particularly 0.01-0.1.

Specifically, the pharmaceutical composition may further comprise one or more pharmaceutically acceptable excipients, such as carriers, adjuvants, and diluents.

The pharmaceutically acceptable excipients are pharmaceutically acceptable excipients for injection, such as isotonic sterile saline (sodium dihydrogen phosphate, disodium hydrogen phosphate, sodium chloride, potassium chloride, calcium chloride, magnesium chloride, etc., or mixtures of the above salts) solutions.

Specifically, the pharmaceutical composition may be administered via any suitable route of administration, such as gastrointestinal administration or parenteral administration (e.g., intravenous, intramuscular, subcutaneous, intraorgan, intranasal, intradermal, instillation, intracerebral, and intrarectal administrations); the drug may be in any dosage form, such as dosage forms for gastrointestinal administration, including, for example, but not limited to, tablets, pills, powders, granules, capsules, lozenges, syrups, liquids, emulsions, suspensions, etc.; dosage forms for parenteral administration, including, for example, dosage forms for injection administration such as injections (e.g., for subcutaneous, intravenous, intramuscular and intraperitoneal injections), dosage forms for respiratory administration such as sprays, aerosols, and power aerosols, dosage forms for skin administration such as external solutions, lotions, ointments, plasters, pastes, and patches, dosage forms for mucosa administration such as eye drops, eye ointments, nose drops, gargles, and sublingual tablets, and dosage forms for oral administration such as suppositories, aerosols, effervescent tablets, drops, and dripping pills, which are used for rectum, vagina, urethra, nasal cavity, auditory canal, etc. Particularly, dosage forms for injection administration are adopted.

Specifically, various dosage forms of the pharmaceutical composition may be prepared according to conventional production methods in the pharmaceutical field.

In a sixth aspect, the present invention provides use of the compound according to the first aspect or a pharmaceutically acceptable salt, an ester, an isomer, a prodrug and a solvate thereof, or the lipid composition according to the fourth aspect in the preparation of a delivery system for a bioactive substance and in the delivery of a bioactive substance.

Specifically, in the use, the bioactive substance has the definition as described in the fourth aspect of the nresent invention

Specifically, the delivery system for the bioactive substance is lipid nanoparticles (LNPs).

In a seventh aspect, the present invention provides use of the compound according to the first aspect or a pharmaceutically acceptable salt, an ester, an isomer, a prodrug and a solvate thereof, the lipid composition according to the fourth aspect, or the pharmaceutical composition in the preparation of a medicament for preventing and/or treating diseases and in the prevention and/or treatment of diseases.

Specifically, the diseases may be selected from one or more of: cancer, inflammation, fibrotic disease, autoimmune disease, disease caused by infection of a pathogen, psychiatric disorder, hematological disorder, chromosomal disease, genetic disease, connective tissue disease, digestive disease, ear-nose-throat disease, endocrine disease, ocular disease, reproductive disease, heart disease, kidney disease, lung disease, metabolic disorder, oral disease, musculoskeletal disease, nutritional disease, skin disease, etc.

Specifically, the pathogen may be a microorganism, a parasite (protozoa, helminth, etc.), or other vectors; wherein the microorganism may be selected from: one or more of viruses, chlamydias, rickettsiae, mycoplasmas, bacteria, spirochetes, fungi, etc.

Specifically, the viruses are, for example, but not limited to, Adenoviridae (e.g., adenovirus), Herpesviridae (e.g., HSV1 (herpes of mouth), HSV2 (herpes of external genitalia), VZV (chicken pox), EBV (Epstein-Barr virus), CMV (cytomegalovirus)), Poxviridae (e.g., smallpox virus, vaccinia virus), Papovavirus (e.g., human papilloma virus (HPV)), Parvoviridae (e.g., B19 virus), Hepadnaviridae (e.g., hepatitis B virus), Polyomaviridae (e.g., polyomavirus), Reoviridae (e.g., reovirus, rotavirus), Picornaviridae (e.g., enterovirus, foot-and-mouth disease virus), Caliciviridae (e.g., Norwalk virus, hepatitis E virus), Togaviridae (e.g., rubella virus), Arenaviridae (e.g., lymphocytic choriomeningitis virus), Retroviridae (HIV-1, HIV-2, HTLV-1), Flaviviridae (e.g., Dengue virus, Zika virus, encephalitis B virus, Chikungunya virus, yellow fever virus, hepatitis C virus, West Nile virus), Orthomyxoviridae (e.g., influenza viruses (e.g., influenza A virus, influenza B virus, influenza C virus)), Paramyxoviridae (e.g., human parainfluenza virus type 1 (HPIV), HPIV type 2, HPIV type 3, HPIV type 4, Sendai virus, mumps virus, measles virus, respiratory syncytial virus, Newcastle disease virus, etc.), Bunyaviridae (e.g., California encephalitis virus, Hantavirus), Rhabdoviridae (e.g., rabies virus), Filoviridae (e.g., Ebola virus, Marburg virus), Coronaviridae (e.g., HCoV-229E, HCoV-OC43, HCoV-NL63, HCoV-HKU1, SARS-CoV, MERS-CoV, SARS-CoV-2), Astroviridae (e.g., astrovirus), and Bornaviridae (e.g., Borna virus) viruses.

In some embodiments of the present invention, the virus is a coronavirus, specifically, for example, HCoV-229E, HCoV-OC43, HCoV-NL63, HCoV-HKU1, SARS-CoV, MERS-CoV, or SARS-CoV-2.

In some embodiments of the present invention, the virus is an orthomyxovirus, for example, an influenza virus (e.g., influenza A virus, influenza B virus, or influenza C virus).

In some embodiments of the present invention, the virus is a paramyxovirus, for example, HPIV type 1, HPIV type 2, HPIV type 3, HPIV type 4, Sendai virus, mumps virus, measles virus, or respiratory syncytial virus.

In some embodiments of the present invention, the virus is a flavivirus, for example, Dengue virus, Zika virus, encephalitis B virus, Chikungunya virus, yellow fever virus, hepatitis C virus, or West Nile virus.

In some embodiments of the present invention, the virus is a human papilloma virus (HPV).

Specifically, the diseases caused by viral infection include, but are not limited to, influenza, SARS, COVID-19, viral hepatitis (e.g., hepatitis A, hepatitis B, hepatitis C, hepatitis D), mumps, measles, Dengue fever, Zika virus disease, encephalitis B virus, Chikungunya disease, yellow fever, West Nile virus disease, cervical cancer, etc.

In some embodiments of the present invention, the medicament is a medicament for preventing diseases, such as a vaccine.

In some embodiments of the present invention, the medicament is a therapeutic agent for treating diseases.

In an eighth aspect, the present invention provides a method for delivering a bioactive substance, which comprises a step of preparing a delivery system for a bioactive substance from the compound according to the first aspect or a pharmaceutically acceptable salt, an ester, an isomer, a prodrug and a solvate thereof, or the lipid composition according to the fourth aspect, and then delivering the bioactive substance to a subject in need thereof through the delivery system.

Specifically, in the method, the bioactive substance has the definition as described in the fourth aspect of the present invention.

Specifically, the bioactive substance is encapsulated in the delivery system.

Specifically, in the method, the subject may be any animal or cell thereof (*in vitro* or *in situ*) that is subjected to the method; more specifically, the subject is a mammal, e.g., a rat, a mouse, a guinea pig, a rabbit, a dog, a monkey, or a human, particularly a human.

In a ninth aspect, the present invention provides a method for preventing and/or treating diseases, which comprises a step of administering to a subject in need thereof the pharmaceutical composition according to the fifth aspect, or a step of preparing a delivery system for a bioactive substance from the compound according to the first aspect or a pharmaceutically acceptable salt, an ester, an isomer, a prodrug and a solvate thereof, or the lipid composition according to the fourth aspect, and then delivering the bioactive substance to a subject in need thereof through the delivery system, or a step of using the method for delivering a bioactive substance according to the eighth aspect.

Specifically, the subject is a mammal, e.g., a rat, a mouse, a guinea pig, a rabbit, a dog, a monkey, or a human, particularly a human.

The lipid nanoparticles (LNPs) prepared from the lipid compound of the present invention have a significant and continuous inhibitory effect on KDR mRNA after delivering anti-KDR siRNA (an siRNA that inhibits VEGFR2 mRNA expression) into cells, which indicates that the lipid compound and the lipid nanoparticles prepared therefrom can effectively deliver a bioactive substance to a target cell and site in a targeted mode, thereby efficiently achieving the pharmacological effect of the bioactive substance; in addition, the lipid compound of the present invention has a singular molecular weight, which is beneficial for controlling differences between batches, improving the stability of finished drugs and reducing immunogenicity, and thus it is expected to be used for the development and application of related drugs.

### BRIEF DESCRIPTION OF THE DRAWINGS

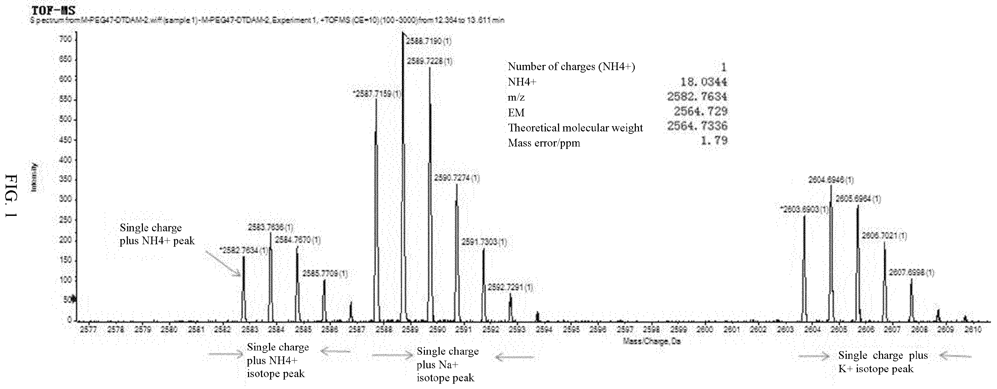

FIG. 1 shows the mass spectrum of compound 1 prepared in Example 1 of the present invention.

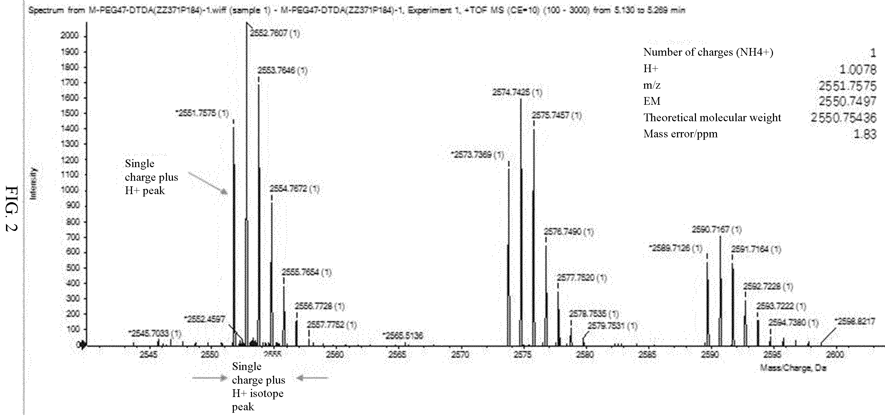

FIG. 2 shows the mass spectrum of compound 2 prepared in Example 2 of the present invention.

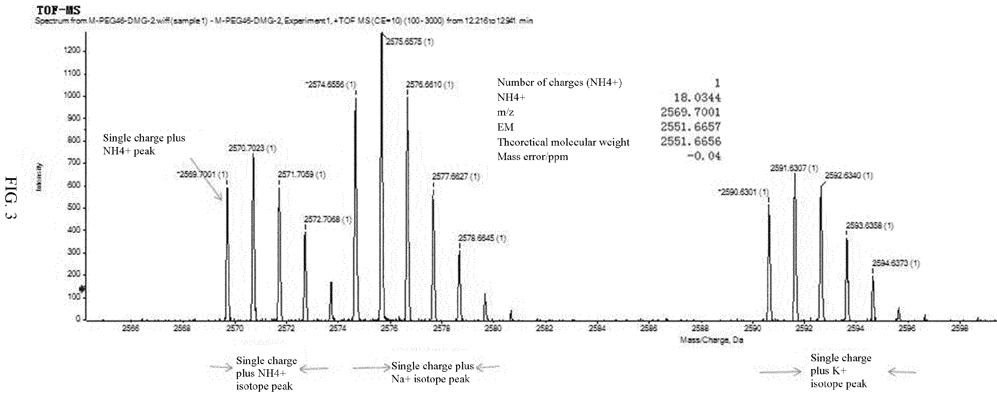

FIG. 3 shows the mass spectrum of compound 3 prepared in Example 3 of the present invention.

### DETAILED DESCRIPTION

Unless otherwise defined, all scientific and technical terms used in the present invention have the same meaning as commonly understood by those skilled in the art to which the present invention relates.

In the present invention, the terms "VEGFR2" and "KDR" both represent the vascular endothelial growth factor receptor 2, and are used interchangeably.

In the present invention, the term "alkyl" refers to a hydrocarbon group that is linear or branched and that does not contain unsaturated bonds, and that is linked to the rest of the molecule via a single bond. The alkyl as used herein typically contains 1 to 10 (e.g., 1, 2, 3, 4, 5, 6, 7, 8, 9, or 10) carbon atoms (i.e., C₁₋₁₀ alkyl), preferably 1 to 6 carbon atoms (i.e., C₁₋₆ alkyl). Examples of alkyl include, but are not limited to, methyl, ethyl, n-propyl, isopropyl, n-butyl, isobutyl, tert-butyl, n-pentyl, isopentyl, neopentyl, tert-pentyl, n-hexyl, isohexyl, and the like. If alkyl is substituted with cycloalkyl, it is correspondingly "cycloalkylalkyl", such as cyclopropylmethyl, cyclopropylethyl, cyclobutylmethyl, cyclopentylmethyl, or cyclohexylmethyl. If alkyl is substituted with aryl, it is correspondingly "aralkyl", such as benzyl, benzhydryl or phenethyl. If alkyl is substituted with heterocyclyl, it is correspondingly "heterocyclylalkyl".

In the present invention, the term "alkylene" refers to a hydrocarbon group (divalent alkyl) formed from an alkane molecule by losing two hydrogen atoms, which may be linear or branched and is linked to other parts of a molecule via a single bond. The alkyl as used herein typically contains 1 to 10 (e.g., 1, 2, 3, 4, 5, 6, 7, 8, 9, or 10) carbon atoms (i.e., C₁₋₁₀ alkylene), particularly 1 to 6 carbon atoms (i.e., C₁₋₆ alkylene). Examples of alkylene include, for example, methylene (-CH₂-), ethylidene (-CH₂CH₂-), propylene, butylene, and the like. In the present invention, the term "alkenyl" refers to a hydrocarbon group that is linear or branched and contains at least two carbon atoms and at least one unsaturated bond, and that is linked to the rest of the molecule via a single bond. The alkenyl as used herein typically contains 1 to 10 (e.g., 1, 2, 3, 4, 5, 6, 7, 8, 9, or 10) carbon atoms (i.e., C₁₋₁₀ alkenyl), preferably 1 to 6 carbon atoms (i.e., C₁₋₆ alkenyl). Examples of alkenyl include, but are not limited to, ethenyl, 1-methyl-ethenyl, 1-propenyl, 2-propenyl, or butenyl, and the like.

In the present invention, the term "cycloalkyl" refers to an alicyclic hydrocarbon, and the cycloalkyl as used herein typically contains 1 to 4 monocyclic and/or fused rings, and 3 to 18 carbon atoms, preferably 3 to 10 (e.g., 3, 4, 5, 6, 7, 8, 9, or 10) carbon atoms (e.g., C₃₋₁₀ cycloalkyl, C₃₋₆ cycloalkyl), such as cyclopropyl, cyclobutyl, cyclopentyl, cyclohexyl, cycloheptyl, cyclooctyl, or adamantyl.

In the present invention, the term "aryl" refers to any functional group or substituent derived from a simple aromatic ring, including monocyclic aryl groups and/or fused aryl groups, such as those containing 1 to 3 monocyclic or fused rings, and having 6 to 18 (e.g., 6, 8, 10, 12, 14, 16, or 18) carbon ring atoms. The aryl as used herein is typically an aryl group that contains 1 to 2 monocyclic or fused rings and has 6 to 12 carbon ring atoms (i.e., C₆₋₁₂ aryl), wherein H on the carbon atoms may be substituted, for example, with alkyl, halogen, and other groups. Examples of aryl include, but are not limited to, phenyl, *p*-methylphenyl, naphthyl, biphenyl, indenyl, and the like.

In the present invention, the term "halogen" refers to bromine, chlorine, iodine, or fluorine.

In the present invention, the term "heterocyclyl" refers to a 3- to 18-membered non-aromatic ring group containing 2 to 17 carbon atoms and 1 to 10 heteroatoms. Heterocyclyl may be a monocyclic, bicyclic, tricyclic or tetracyclic ring system, which may include fused, spiro or bridged ring systems. Heterocyclyl may be partially saturated (heteroaryl) or fully saturated (heterocycloalkyl). Suitable heteroaryl groups for the compound of the present invention contain 1, 2 or 3 heteroatoms selected from N, O and S atoms and include, for example, coumarin, including 8-coumarin, quinolyl, including 8-quinolyl, isoquinolyl, pyridinyl, pyrazinyl, pyrazolyl, pyrimidinyl, furyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuranyl, benzofurazanyl, benzothiophenyl, benzothiazolyl, benzoxazolyl, quinazolinyl, quinoxalinyl, naphthyridinyl, and furopyridinyl. Suitable heterocycloalkyl groups for the compound of the present invention contain 1, 2 or 3 heteroatoms selected from N, O and S atoms and include, for example, pyrrolidinyl, tetrahydrofuryl, dihydrofuran, tetrahydrothienyl, tetrahydrothiopyranyl, piperidinyl, morpholinyl, thiomorpholinyl, oxathianyl, piperazinyl, azetidinyl, oxetanyl, thietanyl, homopiperidinyl, oxiranyl, thiiranyl, azepinyl, oxazepanyl, diazepinyl, triazepinyl, 1,2,3,6-tetrahydropyridinyl, 2-pyrrolinyl, 3-pyrrolinyl, indolinyl, 2*H-*pyranyl, 4*H*-pyranyl, dioxanyl, 1,3-dioxolanyl, pyrazolinyl, dithianyl, dithiolyl, dihydropyranyl, dihydrothienyl, pyrazolidinyl, imidazolinyl, imidazolidinyl, 3-azabicyclo[3.1.0]hexyl, 3-azabicyclo[4.1.0]heptyl, 3*H*-indolyl, and quinolizinyl.

The pharmaceutically acceptable salts of the present invention include acid addition salts and base addition salts.

The acid addition salts include, but are not limited to, salts derived from inorganic acids, such as hydrochloric acid, nitric acid, phosphoric acid, sulfuric acid, hydrobromic acid, hydroiodic acid, and phosphonic acid, and salts derived from organic acids, such as aliphatic mono-carboxylic acid and aliphatic dicarboxylic acid, phenyl-substituted alkanoic acid, hydroxyalkanoic acid, alkanedioic acids, aromatic acid, aliphatic sulfonic acid and aromatic sulfonic acid. Thus, these salts include, but are not limited to, sulfate, pyrosulfate, bisulfate, sulfite, bisulfite, nitrate, phosphate, monohydrogenphosphate, dihydrogenphosphate, metaphosphate, pyrophosphate, hydrochloride, hydrobromide, iodate, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, tosylate, phenylacetate, citrate, lactate, maleate, tartrate, and methanesulfonate, and salts comprising amino acids such as arginate, gluconate and galacturonate. Acid addition salts can be prepared by contacting the free base form with a sufficient amount of the desired acid to form the salt in a conventional manner. The free base form can be regenerated by contacting the salt form with a base and isolating the free base in a conventional manner.

The base addition salts according to the present invention are salts with metals or amines, such as hydroxides of alkali metals and alkaline earth metals, or with organic amines. Examples of metals used as cations include, but are not limited to, sodium, potassium, magnesium and calcium. Examples of suitable amines include, but are not limited to, N,N-dibenzylethylenediamine, chloroprocaine, choline, diethanolamine, ethylenediamine(ethane-1,2-diamine), N-methylglucamine and procaine. Base addition salts can be prepared by contacting the free acid form with a sufficient amount of the desired base to form the salt in a conventional manner. The free acid form can be regenerated by contacting the salt form with an acid and isolating the free acid in a conventional manner.

In the present invention, the stereoisomer includes enantiomeric, diastereomeric and geometric forms. Some of the compounds of the present invention have cyclohydrocarbyl which may be substituted on more than one carbon atom, in which case all geometric forms thereof, including cis and trans, and mixtures thereof, are within the scope of the present invention. The cyclohydrocarbyl includes aliphatic cyclohydrocarbyl and aryl, wherein the alicyclic cyclohydrocarbyl may be non-aromatic, monocyclic, fused, bridged or spiro, saturated or unsaturated cyclic hydrocarbyl, and the aryl may be phenyl, naphthyl, phenanthryl, biphenyl and the like.

In the present invention, the solvate refers to a physical association of the compound of the present invention with one or more solvent molecules. The physical association includes various degrees of ionic and covalent bonding, including hydrogen bonding. In some cases, the solvate can be isolated, for example, when one or more solvent molecules are incorporated into the crystal lattice of a crystalline solid. Solvates include both solution phases and isolatable solvates. Representative solvates include ethanolates, methanolates, and the like.

In the present invention, the term "prodrug" refers to forms of the compound of formula I (including acetals, esters, and zwitterions) which are suitable for administration to patients without undue toxicity, irritation, allergic response and the like, and which are effective for the intended use thereof. The prodrug is converted *in vivo,* e.g., by hydrolysis in blood, to give the parent compound.

In the present invention, the term "nucleic acid" refers to a polymer containing at least two deoxyribonucleotides or ribonucleotides in either single- or double-stranded form and includes DNA, RNA, and hybrids thereof.

In the present invention, the term "lipid" refers to a group of organic compounds that include, but are not limited to, esters of fatty acids and are generally characterized by being poorly soluble in water, but soluble in many organic solvents.

In the present invention, the term "cationic lipid" refers to a lipid molecule capable of being positively charged. In the present invention, the term "neutral lipid" refers to an uncharged, non-phosphoglyceride lipid molecule. In the present invention, the term "lipid nanoparticle" refers to a particle having at least one nanoscale dimension, which comprises at least one lipid.

In the present invention, the term "vaccine" refers to a composition suitable for application to an animal (particularly a mammal, such as a human) that induces an immune response upon administration, with a strength sufficient to help prevent, improve, or cure clinical diseases caused by microbial infections as a minimum.

In the present invention, the term "delivery system" refers to a formulation or composition that regulates the spatial, temporal and dose distribution of a biologically active ingredient in an organism.

In the present invention, the terms "patient" and "subject" are used interchangeably herein and refer to any animal or cell thereof, whether *in vitro* or *in situ,* treated according to the method described herein. Specifically, the aforementioned animal includes mammals, for example, rats, mice, guinea pigs, rabbits, dogs, monkeys, or humans, particularly humans.

In the present invention, the term "treating" refers to preventing, curing, reversing, attenuating, alleviating, minimizing, suppressing, arresting, and/or stopping one or more clinical symptoms of a disease after its onset. In the present invention, the term "preventing" refers to treatment to avoid, minimize, or make difficult the onset or progression of a disease prior to its onset.

The technical solutions of the present invention will be clearly and completely described below with reference to the examples of the present invention, and it is obvious that the described examples are only a part of the examples of the present invention but not all of them. Based on the examples of the present invention, all other examples obtained by those of ordinary skills in the art without creative work shall fall within the protection scope of the present invention.

### Example 1: Synthesis of Compound 1

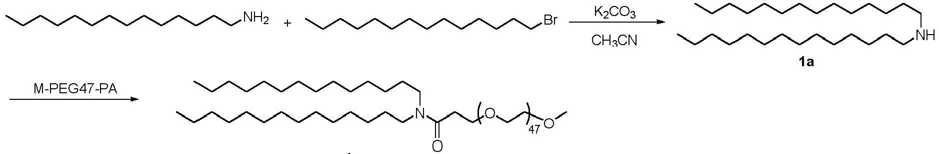

### 1. Synthesis of compound 1a

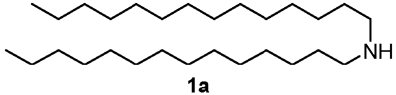

N-tetradecylamine (3.0 g, 14 mmol) and 1-bromotetradecane (3.9 g, 14 mmol) were dissolved in 20 mL of acetonitrile, then potassium carbonate (1.9 g, 14 mmol) was added, and the mixture was stirred at 80 °C for 12 h. After the reaction starting materials were consumed completely, the reaction system was cooled to room temperature and filtered under vacuum, the filter residue was washed with dichloromethane, and a saturated sodium bicarbonate solution was added to the resulting filtrate, followed by extraction with dichloromethane for 2 times. The organic phases were combined, dried over anhydrous sodium sulfate, filtered and concentrated, and the residue was separated by column chromatography to give compound 1a (3.9 g, white solid) in a yield of 68%. MS m/z (ESI): 410.40 [M+1].

### 2. Synthesis of compound 1

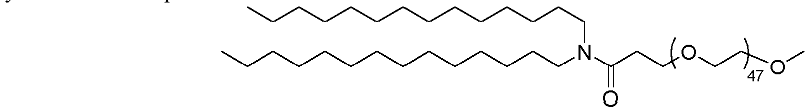

Compound 1a (0.576 g, 1.4 mmol) and 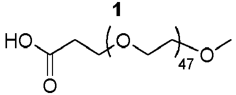 (2 g, 1 mmol) were added to a 100-mL single-necked flask containing dichloromethane (20 mL) for dissolution with stirring, then DIEA (260 mg, 2 mmol) and HATU (760 mg, 2 mmol) were sequentially added, and the mixture was stirred at room temperature for 5 h. The reaction system was filtered, the mother liquor was concentrated under vacuum, and then 40 mL of purified water was added for dissolution with stirring. The system was washed with ethyl acetate (40 mL × 3) (the system would be emulsified in the washing process, and thus a small amount of ethanol was added for demulsification). After washing, 6 g of sodium chloride was added to the aqueous phase for dissolution with stirring, and the aqueous phase was extracted with dichloromethane (20 mL × 2). The organic phases were combined, dried over anhydrous sodium sulfate, and filtered under vacuum. The filtrate was concentrated under vacuum, 30 mL of isopropanol and 10 mL of ethyl acetate were added, and the reaction system was heated to 40 °C for dissolution, then cooled to -15 °C for crystallization, and filtered under vacuum. The filter cake was dried under vacuum to give the product compound 1 (1.5 g, white solid) in a yield of 62.5%. MS m/z (ESI): 2582.76 [M+NH₄]⁺;
1H-NMR (300 MHz, DMSO) δ: 2.96 (2H, s), 3.1-3.8 (180H, m), 1.46 (4H, m), 1.24 (44H, m), 0.86 (6H, t).

### Example 2: Synthesis of Compound 2

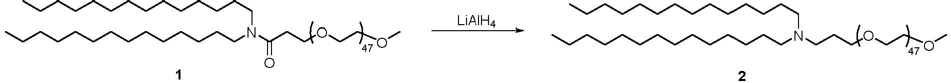

LiAlH₄ (380 mg, 10 mmol) was slowly added to a three-necked flask containing 30 mL of tetrahydrofuran in an ice-water bath under nitrogen atmosphere. Compound 1 (2 g, 1 mmol) was dissolved in 10 mL of tetrahydrofuran, and the reaction solution was slowly and dropwise added to a reaction flask; 10 mL of purified water was dropwise added to the reaction system after 3 h, the system was filtered through celite, and the filtrate was concentrated under vacuum; 20 mL of isopropanol and 6 mL of ethyl acetate were added, and the system was heated to 40 °C for dissolution, then cooled in an ice-water bath for crystallization, and filtered under vacuum. The filter cake was dried under vacuum to give the product compound 2 (1.3g, white solid) in a yield of 64%. MS m/z (ESI): 2551.76 [M+H]⁺;
1H-NMR (300 MHz, DMSO) δ: 3.2-3.8 (180H, m), 2.36 (4H, t), 2.26 (4H, m), 1.53 (2H, m), 1.22 (44H, m), 0.84 (6H, t).

### Example 3: Synthesis of Compound 3

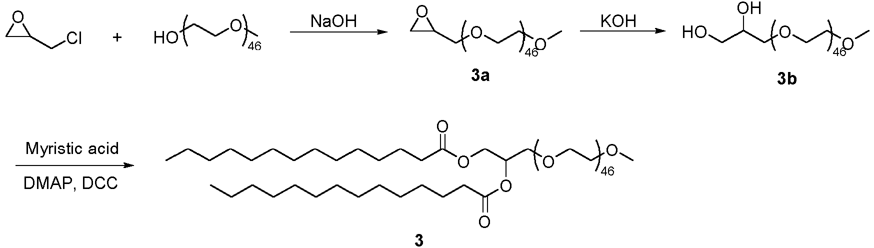

### 1. Synthesis of compound 3a

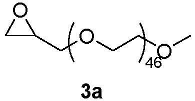

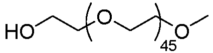

(40 g, 20 mmol) and purified water (0.4 ml) were added to a 1-L three-necked flask containing THF (400 mL) for dissolution with stirring, then NaOH (6.4 g, 160 mmol) and epichlorohydrin (37 g, 400 mmol) were sequentially added, and the mixture was heated and refluxed for 18 h. NaOH (3.2 g, 80 mmol) and epichlorohydrin (18.5 g, 200 mmol) were supplemented, and then the system was reacted for 18 h. The reaction system was cooled to room temperature and filtered through celite, then a phosphate (100 mL) buffer (pH = 7) was added to the mother liquor, and the system was concentrated under vacuum to remove THF. After concentration, purified water (200 mL) was added, and the system was washed with ethyl acetate (100 mL × 2). After washing, 45 g of sodium chloride was added to the aqueous phase for dissolution with stirring, and the aqueous phase was extracted with dichloromethane (100 mL × 2). The organic phases were combined, dried over anhydrous sodium sulfate, and filtered under vacuum, the filtrate was concentrated under vacuum, and then 400 mL of isopropanol was added for recrystallization. The residue was filtered under vacuum, and the filter cake was dried under vacuum to give the product compound 3a (34 g, light yellow solid) in a yield of 85%. MS m/z (ESI): 2114.41 [M+H]⁺;
1H-NMR (300 MHz, DMSO) δ: 3.3-3.85 (184H, m), 3.28 (1H, t), 3.28 (3H, s), 3.1 (1H, m), 2.7 (1H, t), 2,55 (1H, m).

### 2. Synthesis of compound 3b

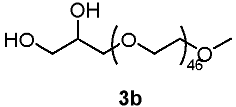

Compound 3a (30 g, 15 mmol) was dissolved in a 2 mol/L potassium hydroxide solution (300 mL) with stirring at room temperature for 12 h, then 45 g of sodium chloride was added for dissolution with stirring, and the aqueous phase was extracted with dichloromethane (150 mL × 2). The organic phases were combined, dried over anhydrous sodium sulfate, and filtered under vacuum, the filtrate was concentrated under vacuum, and then 450 mL of methyl *tert*-butyl ether was added to precipitate a solid. The residue was filtered under vacuum, and the filter cake was dried under vacuum to give the product compound 3b (28 g, light yellow solid) in a yield of 93.3%. MS m/z (ESI): 2132.43 [M+H]⁺;
1H-NMR (300 MHz, DMSO) δ: 4.6 (1H, s), 4.45 (1H, s), 3.3-3.8 (189H, m), 3.25 (3H, s).

### 3. Synthesis of compound 3

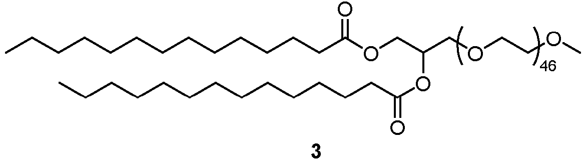

Compound 3b (20 g, 10 mmol) was added to a 500-mL single-necked flask containing DCM (200 mL) for dissolution with stirring, then tetradecanoic acid (6.4 g, 28 mmol) and DMAP (489 mg, 4 mmol) were sequentially added, and the mixture was placed in an ice-water bath. DCC (5.8 g, 28 mmol) was dissolved in 40 mL of DCM, and the resulting solution was added dropwise to the reaction system. After 16 h, the reaction system was filtered, then the mother liquor was concentrated under vacuum to remove dichloromethane, and 300 mL of isopropanol and 100 mL of ethyl acetate were added. The system was heated to 40 °C for dissolution, then cooled to -10 °C for crystallization, and filtered under vacuum. The filter cake was dried under vacuum to give the product compound 3 (17 g, light yellow solid) in a yield of 85%. MS m/z (ESI): 2569.70 [M+NH₄]⁺;
1H-NMR (300 MHz, DMSO) δ: 5.1 (1H, s), 4.28 (1H, m), 4.1 (1H, m), 3.3-3.8 (183H, m), 3.24 (3H, s), 2.25 (4H, t), 1.5 (4H, m), 1.24 (44H, m), 0.86 (6H, t).

### Example 4: Gene Inhibitory Effect of siRNA-Polyethylene Glycol Lipid (PEG-Lipid) Particle Composition After Cell Delivery in ARPE-19 Cells

### 1. Cell culture and transfection

### Cell name: ARPE-19

(1) ARPE-19 cells were cultured in a DMEM/F12 + 12% FBS + double antibody medium (containing 100 U/mL Penicillin, 100 µg/mL Streptomycin) and placed in an incubator at 37 °C with 5% CO₂ and saturated humidity. 24 h before the experiment, the cells were seeded onto a 12-well plate at 1.2 × 10⁵ cell/well and cultured overnight.
(2) 50 µL of diluted anti-KDR siRNA:compound 1 (1:10), anti-KDR siRNA:compound 2 (1:10), and anti-KDR siRNA:compound 3 (1:10) (in molar ratios; the siRNA concentration was 20 µM) from 2.5 mL with an OPTI-MEM culture medium were separately mixed with 50 µL of a diluted Lipofectamine 3000^{™} transfection reagent from 3 µL with an OPTI-MEM culture medium for positive control, followed by gently shaking, and the mixed solutions were left to stand for 15 min. In addition, a blank group, an NC group, and an NC-RL group were set.
   Compound 1, compound 2 and compound 3 were prepared in Examples 1-3, respectively.
   Anti-KDR siRNA is an siRNA that inhibits VEGFR2 mRNA expression, and the preparation method therefor is described in patent document CN202010229195.2; the sequence is as follows:
   Sense strand: 5'-GGAGUGAGAUGAAGAAAUU-3';
   Antisense strand: 5'-AAUUUCUUCAUCUCACUCC-3'.
(3) The medium was refreshed for the cells in each well in the cell plate, an antibiotic-free culture medium was added at 900 µL/well, and then 100 µL of the mixed solution was added into each well, finally the total volume of each well being 1000 µL. The siRNA (or siRNA NC, or siR-NC RL) transfection concentration was 50 nM.
(4) 24 h and 48 h after transfection, the 12-well plate was taken out from the incubator at 37 °C with 5% CO₂, and the cells were harvested for RNA extraction for subsequent detection.

### 2. RNA extraction

(1) RNA was extracted using an RNA extraction kit from Promega. Briefly, after the cells were washed with PBS, 300 µL of lysis solution was added, and the cells were pipetted using a pipettor. After full lysis, 300 µL of diluent was added, and then the mixture was placed in a water bath and reacted at 70 °C for 3 min. The mixture was centrifuged at 14000 rpm for 10 min, the supernatant was transferred to a new 1.5 mL EP tube, and then 300 µL of absolute ethanol was added. The resulting mixture was uniformly mixed and added into an adsorption column. The mixture was centrifuged at 14000 rpm for 1 min, the filtrate was discarded, 600 µL of washing solution was then added, and the resulting mixture was centrifuged again for 1 min. The filtrate was discarded, the prepared 50 µL DNA enzyme reaction solution was then added into each well, and the mixture was left to stand at room temperature for 15 min; 600 µL of washing solution was added, and the mixture was centrifuged for 1 min; after the filtrate was discarded, 600 µL of washing solution was added for centrifugation for 1 min again; the filtrate was discarded, the residue was centrifuged for 2 min, 50 µL of nuclease-free water was then added into each well, and the mixture was left to stand at room temperature for 5 min and then centrifuged to collect eluted RNA.
(2) Quality control of RNA: RNA content and purity were detected by Nanodrop, and RNA integrity was detected by 1% agarose gel electrophoresis.

### 3. Q-PCR detecting procedure

### (1) Reverse transcription of RNA

Total RNA extracted from the sample was used as a template; using a Promega reverse transcription kit, a reaction system was established as follows:

**Table 1. RNA reverse transcription system**

| Reagent | Amount |
|---|---|
| 5×RT Buffer(oligodT) | 4 µL |
| RTase Mix | 2 µL |
| RNA template | 1 µg |
| RNase-free H₂O | Supplement to 20 µL |
| Total | 20 µL |

The system was well mixed and centrifuged to allow the liquid to be all at the bottom of the tube; the reverse transcription was performed at 42 °C for 60 min and 72 °C for 10 min, and the product was the cDNA template.

### (2) Quantitative fluorescence PCR detection

Using a TB green Premix Ex Taq II (Tli RNaseH Plus) (Promega) reagent, a reaction system was established as follows:

**Table 2. Quantitative fluorescence PCR reaction system**

| Reagent | Amount |
|---|---|
| 2×SYBR Green Mix | 10 µL |
| Forward primer (10 µM) | 0.4 µL |
| Reverse primer (10 µM) | 0.4 µL |
| cDNA | 2 µL |
| RNase-free H₂O | 7.2 µL |
| Total | 20 µL |

PCR amplification was performed according to the procedure as follows:
Pre-denaturation at 95 °C for 10min, followed by the cycle as follows:
*95 °C 10 s
60 °C 20 s
70°C 10 s

### Plate reading

Return to * for 40 cycles in total.

Plot a melting curve: read the plate every 0.5 °C and then stop for 5 s between 65 °C and 95 °C.

### 4. Gene inhibitory effect

The relative expression level of KDR mRNA was calculated by ΔΔCt with GAPDH as an endogenous reference gene; mRNA expression levels were normalized for each group with the expression level for the blank group as 100%.

The results show that the relative expression level of KDR mRNA in siRNA-treated cells herein decreased significantly at 24 h after transfection relative to the blank group, the siNC group and the siNC RL group, and further decreased at 48 h after transfection, which indicates that particles composed of compounds 1-3 and siRNA have a significant and continuous inhibitory effect on KDR mRNA.

Example 5: Gene Inhibitory Effect of siRNA-PEG-Lipid Particle Composition After Cell Delivery in A375 Cells

The foregoing procedure was similar to that of Example 4 except that the culture medium for the A375 cells was a DMEM culture medium containing 10% FBS (containing 100 U/mL Penicillin, 100 µg/mL of Streptomycin). The results show that the relative expression level of KDR mRNA in siRNA-treated cells herein decreased significantly at 24 h after transfection relative to the blank group, the siNC group and the siNC RL group, and further decreased at 48 h after transfection, which indicates that particles (compound 1, compound 2, compound 3) prepared from PEG-lipids and siRNA have a significant and continuous inhibitory effect on KDR mRNA.

### Example 6: Gene Inhibitory Effect of PEG-Lipid/Cationic Lipid/Neutral Lipid/Steroidal Lipid-siRNA Nanoparticles (LNPs) After Cell Delivery in ARPE-19 Cells

The formula and preparation method for PEG-lipid/cationic lipid/neutral lipid/steroidal lipid-siRNA nanoparticles (LNPs) in each group are as follows:
The compound 1/ALC-0315/DSPC/cholesterol was dissolved in absolute ethanol at a molar ratio of 46.3%/1.7%/9.4%/42.6%, respectively, to prepare a 10 mmol/L mixed solution, and then a citric acid buffer (pH = 4) was added to prepare a 30% ethanol-citric acid solution containing four lipids, which was filtered through a 0.1 µm filter membrane for later use. Anti-KDR siRNA was dissolved in a lipid-free 30% ethanol-citric acid solution at a concentration of 2 mg/mL, and then mixed with the 30% ethanol-citric acid solution containing four lipids according to a mass ratio of anti-KDR siRNA to lipid of 0.06:1, and the mixture was incubated for 30 min, dialyzed with PBS (pH = 7.4) for more than 16 h, and freeze-dried to obtain lipid nanoparticles containing anti-KDR siRNA. The method for preparing different PEG-lipid nanoparticles of other groups (compound 1 was replaced by compound 2 or compound 3) was the same as that for compound 1.

The results show that after the lipid nanoparticles of each group prepared from PEG-lipid/ALC-0315/DSPC/cholesterol and siRNA delivered anti-KDR siRNA into cells, the lipid nanoparticles had a significant and continuous inhibitory effect on KDR mRNA.

### Example 7: Gene Inhibitory Effect of PEG-Lipid/Cationic Lipid/Neutral Lipid/Steroidal Lipid-mRNA Nanoparticles After Cell Delivery in 293T Cells

The formula and preparation method for PEG-lipid/cationic lipid/neutral lipid/steroidal lipid-siRNA nanoparticles (LNPs) of each group are as follows:
The compound 1/ALC-0315/DSPC/cholesterol was dissolved in absolute ethanol at a molar ratio of 46.3%/1.7%/9.4%/42.6%, respectively, to prepare a 10 mmol/L mixed solution, and then a citric acid buffer (pH = 4) was added to prepare a 30% ethanol-citric acid solution containing four lipids, which was filtered through a 0.1 µm filter membrane for later use. GFP mRNA was dissolved in a lipid-free 30% ethanol-citric acid solution at a concentration of 2 mg/mL, and then mixed with the 30% ethanol-citric acid solution containing four lipids according to a mass ratio of GFP mRNA to lipid of 0.06:1, and the mixture was incubated for 30 min, dialyzed with PBS (pH = 7.4) for more than 16 h, and freeze-dried to obtain lipid nanoparticles containing GFP mRNA. The method for preparing different cationic lipid nanoparticles of other groups (compound 1 was replaced by compound 2 or compound 3) was the same as that for compound 1.

The transfection effect of LNPs was determined by detecting the number of cells expressing green fluorescent protein (GFP) using a fluorescence microscope, and the results show that compounds 1-3 had a good delivery effect as the LNPs groups of PEG lipids.

### Example 8: Allergy Study of PEG-Lipids with Different Molecular Weights in Guinea Pigs

### 1. Animal grouping

48 healthy guinea pigs which were fed with food normally for 5 days were randomly divided into 6 groups, with 8 guinea pigs for each group. Group A: negative control group (0.9% sodium chloride injection); group B: positive control group (0.15 mg/mL ovalbumin solution, available from Sigma, USA, lot No. DHO15-4); group C: M-PEG47-DTDPAM (i.e., compound 1, prepared in Example 1); group D: M-PEG47-DTDPA (i.e., compound 2, prepared in Example 2); group E: M-DTDPAM-2000 (available from Tianjin JenKem Technology Co., Ltd., lot No. ZZ409P002); group F: M-DTDPA-2000 (available from Tianjin JenKem Technology Co., Ltd., lot No. ZZ409P004).

### 2. Method of administration

Sensitization was carried out by intraperitoneal injection, and the administration was carried out once every other day at a volume of 1 mL/guinea pig for 4 times in total. On the second day after the last sensitization, each group of guinea pigs was challenged by applying a corresponding liquid medicine to the toes thereof via intravenous injection at a dose of 2 mL/guinea pig. The animal state was observed daily during sensitization, the reaction of guinea pigs was continuously observed for 40 min at 15 min before and after challenging via intravenous injection, and the time of onset and disappearance of symptoms was recorded. Blood was collected 40 min after challenging and drug withdrawal, anticoagulated with heparin, and made into blood plasma.

### 3. Observation index

During sensitization and at the time of challenge administration, animals were observed and recorded for the onset and duration of symptoms, such as nasal scratching, sneezing, restlessness, jumping, panting, and purpura, and the score and grade of symptoms were determined according to the criteria for grading symptoms of systemic sensitization (Table 3).

**Table 3. Criteria for grading symptoms of systemic sensitization**

| Allergy | Scoring | Symptom | Grade |
|---|---|---|---|
| - | 0 | Normal | Negative |
| + | 1 | Restlessness, piloerection, trembling and nose scratching | Weakly positive |
| ++ | 2 | Sneezing, coughing, shortness of breath, urination, defecation, and lacrimation | Positive |
| +++ | 3 | Difficulty in breathing, wheezing, purpura, gait instability, jumping, panting, cramping, whirling, and periodic respiration | Strongly positive |
| ++++ | 4 | Dead | Extremely positive |

### 4. Experimental results

### (1) Systemic allergy experimental results

Guinea pigs of each group exhibited no abnormal symptoms at the time of first sensitization. During sensitization, no abnormality was shown in diet, water drinking and behavior, and the body weight increased normally. Animals of group A had no allergies after challenge administration; animals of group B had a strong allergy after challenge administration, shown as spasticity, purpura, gait instability, panting, jumping, lacrimation, and listlessness; 3 guinea pigs of group C had occasional symptoms such as scratching and restlessness 10-35 min after challenge administration; 2 animals of group D had the above symptoms as those of group C. In group E, 4 guinea pigs had symptoms such as sneezing, coughing, and tachypnea, and 1 guinea pig had symptoms such as piloerection and trembling; in group F, 3 guinea pigs had symptoms such as sneezing, coughing, and shortness of breath, and 2 guinea pigs had symptoms such as trembling and nose scratching; no other guinea pigs were abnormal. The grade of symptoms of the guinea pigs in each group was determined according to Table 3, wherein group B was extremely positive, groups C and D were weakly positive, and groups E and F were positive. However, the number of onset and degree of onset of allergies in groups C and D were lower than those in groups E and F. It was suggested that PEG-lipids of a single molecular weight were less allergic to guinea pigs than PEG-lipids of a non-single molecular weight. The results of grading the symptoms of groups of guinea pigs after the challenge administration are shown in Table 4.

**Table 4. Results of allergies in guinea pigs due to PEG-lipids of different molecular weights**

| Code | Groups | Mean score | Allergic symptoms |
|---|---|---|---|
| Group A | Negative control group | 0 | Negative |
| Group B | Positive control group | 2.8 | Strongly positive |
| Group C | M-PEG47-DTDPAM | 0.375 | Weakly positive |
| Group D | M-PEG47-DTDPA | 0.25 | Weakly positive |
| Group E | M-DTDPAM-2000 | 1.125 | Positive |
| Group F | M-DTDPA-2000 | 1 | Positive |

### (2) Comparison of plasma IgE and histamine levels

After the challenge administration was carried out for each group of animals, the level of plasma IgE of other groups was higher than that of group A, and the increase degree was lower than 100%. The level of plasma IgE of groups C and D was significantly lower than that of groups E and F.

The levels of plasma and histamine of each administration group were higher than those of group A, wherein the histamine level of group B was significantly different from that of group A (P < 0.05); the levels of plasma and histamine of groups E and F were significantly higher than those of group A, with a statistical difference. While the levels of plasma and histamine increased in groups C and D, they were much lower than those in groups E and F, with no statistical difference compared to group A.

**Table 5. Levels of IgE and histamine in plasma (ng/mL) after challenge administration in groups of guinea**

| Code | Groups | IgE level (ng/ml) | Histamine level (ng/ml) |
|---|---|---|---|
| Group A | Negative control group | 204.83±18.65 | 4.92±0.98 |
| Group B | Positive control group | 286.33±34.67 | 12.21±4.25 |
| Group C | M-PEG47-DTDPAM | 235±26.79 | 5.23±1.21 |
| Group D | M-PEG47-DTDPA | 225±31.44 | 6.71±2.10 |
| Group E | M-DTDPAM-2000 | 255±25.78 | 8.89±2.54 |
| Group F | M-DTDPA-2000 | 248±34.11 | 8.23±2.23 |

| | | | |
|---|---|---|---|
| Conclusion: the systemic allergy experimental results of guinea pigs show that PEG-lipids of a non-single molecular weight were positive, while PEG-lipids of a single molecular weight were weakly positive, which indicates that the immunogenicity of PEG-lipids of a single molecular weight was reduced compared with that of PEG-lipids of a non-single molecular weight. The detection results of plasma IgE and histamine levels also support this conclusion. | | | |

The above description is only for the purpose of illustrating the preferred examples of the present invention, and is not intended to limit the scope of the present invention. Any modifications, made without departing from the principle of the present invention shall fall in the protection scope of the present invention.

The certain order in which the steps of the method are listed in the present invention does not constitute any limitation on the order of the steps of the method.

## Claims

1. A compound having the following structure: 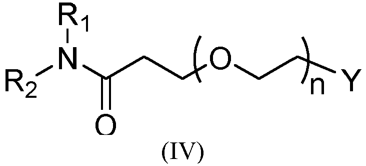 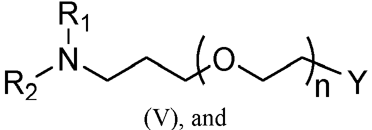 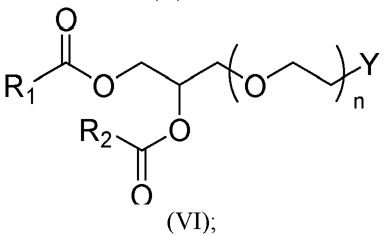
wherein, n is an integer of 30 to 90,
R₁ and R₂ are each independently hydrocarbyl containing 6 to 30 carbon atoms, Y is a terminal group.

2. The compound according to claim 1, wherein the compound has the following structure: 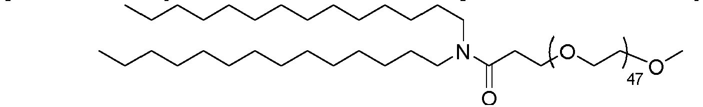 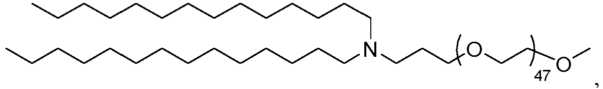 or

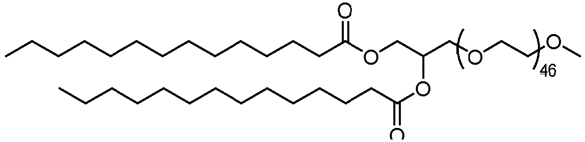

3. A lipid composition comprising the compound according to any one of claims 1 or 2, or a pharmaceutically acceptable salt, an ester, an isomer, a prodrug and a solvate thereof.

4. The composition according to claim 3, further comprising a cationic lipid.

5. The composition according to claim 4, wherein the cationic lipid is selected from: one or more of stearamide (SA), lauryltrimethylammonium bromide, hexadecyltrimethylammonium bromide, myristyltrimethylammonium bromide, dimethyldioctadecylammonium bromide (DDAB), 3β-[*N-*(*N*',*N*'-dimethylaminomethane)-carbamoyl]cholesterol (DC-cholesterol), 1,2-ditetradecanoyl-3-trimethylammonium-propane (DMTAP), 1,2-dioctadecyl-3-trimethylammonium-propane (DOTAP) and DOTAP derivatives such as 1,2-di-(9Z-octadecenoyl)-3-trimethylammoniumpropane and 1,2-dihexadecanoyl-3-trimethylammonium-propane, 1,2-di-(9Z-octadecenoyl)-3-dimethylammonium-propane (DODAP) and DODAP derivatives such as 1,2-ditetradecanoyl-3-dimethylammonium-propane, 1,2-dihexadecanoyl-3-dimethylammonium-propane and 1,2-dioctadecyl-3-dimethylammonium-propane, 1,2-di-O-octadecenyl-3-trimethylammonium propane (DOTMA), 1,2-dioleoyl-c-(4'-trimethylammonium)-butyryl-sn-glycerol (DOTB), dioctadecylamidoalanyl spermine, SAINT-2, a polycationic lipid 2,3-dioleoyloxy-N-[2(sperminecarboxamido)ethyl]-*N,N*-dimethyl-1-propanaminium trifluoroacetate (DOSPA), and ((4-hydroxybutyl)azadialkyl)bis(hexane-6,1-diyl)bis(2-hexyldecanoate) (ALC-0315), particularly ALC-0315.

6. The composition according to claim 4, wherein the molar ratio of the compound to the cationic lipid is 1:0.01-0.1.

7. The composition according to claim 3 to 6, wherein the composition further comprises a neutral lipid.

8. The composition according to claim7, wherein the neutral lipid is selected from: one or more of 1,2-distearoyl-sn-glycero-3-phosphocholine (DSPC), 1,2-dipalmitoyl-sn-glycero-3-phosphocholine (DPPC), 1,2-dioleoyl-sn-glycero-3-phosphoethanolamine (DOPE), 1,2-dipalmitoyl-sn-glycero-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoyl phosphatidylcholine (POPC), and 1-palmitoyl-2-oleoyl phosphatidylethanolamine (POPE), particularly DSPC.

9. The composition according to claim7, wherein the molar ratio of the compound to the neutral lipid is 1:0.1-0.5.

10. The composition according to any one of claims 3 to 9, wherein the composition further comprises a steroidal lipid.

11. The composition according to claim 10, wherein the steroidal lipid is selected from: avenasterol, β-sitosterol, brassicasterol, ergocalciferol, campesterol, cholestanol, cholesterol, coprosterol, dehydrocholesterol, desmosterol, dihydroergocalciferol, dihydrocholesterol, dihydroergosterol, campesterol, epicholesterol, ergosterol, fucosterol, hexahydrosterol, hydroxycholesterol, lanosterol, photosterol, fucasterol, sitostanol, sitosterol, stigmastanol, stigmasterol, cholic acid, glycocholic acid, taurocholic acid, deoxycholic acid, and lithocholic acid, particularly cholesterol.

12. The composition according to claim 10, wherein the molar ratio of the compound to the steroidal lipid is 1:0.5-1.5.

13. The composition according to claim 3, comprising the compound, and ALC-0315, DSPC, and cholesterol.

14. Use of the compound, or the pharmaceutically acceptable salt, the ester, the isomer, the prodrug and the solvate thereof according to any one of claims 1 or 2, or the lipid composition according to any one of claims 3 to 13, in the preparation of a delivery system for a bioactive substance.

15. The use according to claim 14, wherein the bioactive substance is a small molecule compound, a nucleic acid, a peptide, or a protein.

16. The use according to claim 15, wherein the bioactive substance is a nucleic acid, such as DNA or RNA.

17. The use according to claim 15, wherein the RNA is selected from: one or more of an antisense RNA, an saRNA, an mRNA, a lncRNA, an miRNA, an siRNA, a piRNA, a gRNA and a tsRNA, particularly an mRNA or an siRNA.

18. The use according to claim 14 to 17, wherein the delivery system for the bioactive substance is lipid nanoparticles (LNPs).

19. Use of the compound, or the pharmaceutically acceptable salt, the ester, the isomer, the prodrug and the solvate thereof according to any one of claims 1 or 2, or the lipid composition according to any one of claims 3 to 13, in the preparation of a medicament for preventing and/or treating diseases, wherein the diseases are selected from one or more of: cancer, inflammation, fibrotic disease, autoimmune disease, disease caused by infection of a pathogen, psychiatric disorder, hematological disorder, chromosomal disease, genetic disease, connective tissue disease, digestive disease, ear-nose-throat disease, endocrine disease, ocular disease, reproductive disease, heart disease, kidney disease, lung disease, metabolic disorder, oral disease, musculoskeletal disease, nutritional disease, and skin disease.

20. The use according to claim 19, wherein the pathogen is a virus selected from: Adenoviridae, Herpesviridae, Poxviridae, Papovaviridae, Parvoviridae, Hepadnaviridae, Polyomaviridae, Reoviridae, Picornaviridae, Caliciviridae, Togaviridae, Arenaviridae, Retroviridae, Flaviviridae, Orthomyxoviridae, Paramyxoviridae, Bunyaviridae, Rhabdoviridae, Filoviridae, Coronaviridae, Astroviridae, and Bornaviridae viruses.

21. The use according to claim 19, wherein the virus is selected from: HCoV-229E, HCoV-OC43, HCoV-NL63, HCoV-HKU1, SARS-CoV, MERS-CoV, SARS-CoV-2, influenza virus, HPIV type 1, HPIV type 2, HPIV type 3, HPIV type 4, Sendai virus, mumps virus, measles virus, respiratory syncytial virus, Dengue virus, Zika virus, encephalitis B virus, Chikungunya virus, yellow fever virus, hepatitis C virus, West Nile virus, and HPV.

## Patentansprüche

1. Verbindung mit der folgenden Struktur: 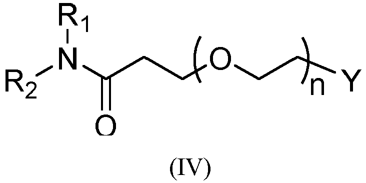 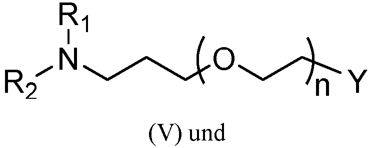 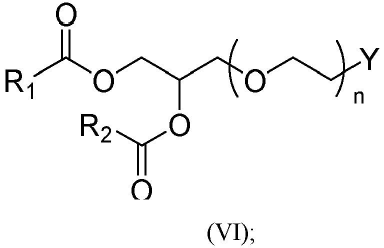
wobei n eine ganze Zahl von 30 bis 90 ist,
R₁ und R₂ jeweils unabhängig Hydrocarbyl sind, das 6 bis 30 Kohlenstoffatome enthält, Y eine endständige Gruppe ist.

2. Verbindung nach Anspruch 1, wobei die Verbindung die folgende Struktur aufweist: 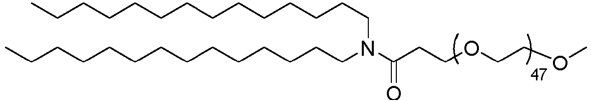 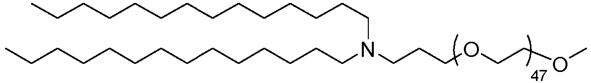 oder

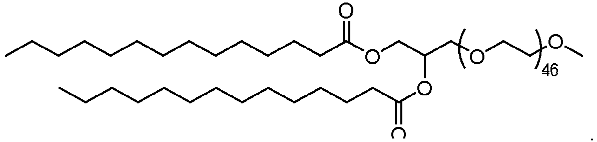

3. Lipidzusammensetzung, umfassend die Verbindung nach einem der Ansprüche 1 oder 2 oder ein pharmazeutisch unbedenkliches Salz, einen Ester, ein Isomer, ein Prodrug und ein Solvat davon.

4. Zusammensetzung nach Anspruch 3, ferner umfassend ein kationisches Lipid.

5. Zusammensetzung nach Anspruch 4, wobei das kationische Lipid ausgewählt ist aus: einem oder mehreren von Stearamid (SA), Lauryltrimethylammoniumbromid, Hexadecyltrimethylammoniumbromid, Myristyltrimethylammoniumbromid, Dimethyldioctadecylammoniumbromid (DDAB), 3β-[*N*-(*N*',*N*'-Dimethylaminoethan)-carbamoyl]cholesterol (DC-Cholesterol), 1,2-Ditetradecanoyl-3-trimethylammonium-propan (DMTAP), 1,2-Dioctadecyl-3-trimethylammonium-propan (DOTAP) und DOTAP-Derivaten wie etwa 1,2-Di-(9Z-octadecenoyl)-3-trimethylammonium-propan und 1,2-Dihexadecanoyl-3-trimethylammonium-propan, 1,2-Di-(9Z-octadecenoyl)-3-dimethylammonium-propan (DODAP) und DODAP-Derivaten wie etwa 1,2-Ditetradecanoyl-3-dimethylammonium-propan, 1,2-Dihexadecanoyl-3-dimethylammonium-propan und 1,2-Dioctadecyl-3-dimethylammoniumpropan, 1,2-Di-O-octadecenyl-3-trimethylammoniumpropan (DOTMA), 1,2-Dioleoyl-c-(4'-trimethylammonium)-butyryl-sn-glycerol (DOTB), Dioctadecylamidoalanylspermin, SAINT-2, einem polykationischen Lipid 2,3-Dioleoyloxy-*N*-[2(spermin-carboxamido)ethyl]-*N*,*N*-dimethyl-1-propanaminiumtrifluoracetat (DOSPA) und ((4-Hydroxybutyl)azadialkyl)bis(hexan-6,1-diyl)bis(2-hexyldecanoat) (ALC-0315), insbesondere ALC-0315.

6. Zusammensetzung nach Anspruch 4, wobei das Molverhältnis der Verbindung zu dem kationischen Lipid 1:0,01-0,1 beträgt.

7. Zusammensetzung nach Anspruch 3 bis 6, wobei die Zusammensetzung ferner ein neutrales Lipid umfasst.

8. Zusammensetzung nach Anspruch 7, wobei das neutrale Lipid ausgewählt ist aus: einem oder mehreren von 1,2-Distearoyl-sn-glycero-3-phosphocholin (DSPC), 1,2-Dipalmitoyl-snglycero-3-phosphocholin (DPPC), 1,2-Dioleoyl-sn-glycero-3-phosphoethanolamin (DOPE), 1,2-Dipalmitoyl-sn-glycero-3-phosphoethanolamin (DPPE), 1,2-Dimyristoyl-sn-glycero-3-phosphoethanolamin (DMPE), 2-Dioleoyl-sn-glycero-3-phospho-(1'-rac-glycerol) (DOPG), Oleoylphosphatidylcholin (POPC) und 1-Palmitoyl-2-oleoylphosphatidylethanolamin (POPE), insbesondere DSPC.

9. Zusammensetzung nach Anspruch 7, wobei das Molverhältnis der Verbindung zu dem neutralen Lipid 1:0,1-0,5 beträgt.

10. Zusammensetzung nach einem der Ansprüche 3 bis 9, wobei die Zusammensetzung ferner ein steroidales Lipid umfasst.

11. Zusammensetzung nach Anspruch 10, wobei das steroidale Lipid ausgewählt ist aus: Avenasterol, β-Sitosterol, Brassicasterol, Ergocalciferol, Campesterol, Cholestanol, Cholesterol, Coprosterol, Dehydrocholesterol, Desmosterol, Dihydroergocalciferol, Dihydrocholesterol, Dihydroergosterol, Campesterol, Epicholesterol, Ergosterol, Fucosterol, Hexahydrosterol, Hydroxycholesterol, Lanosterol, Photosterol, Fucasterol, Sitostanol, Sitosterol, Stigmastanol, Stigmasterol, Cholsäure, Glycocholsäure, Taurocholsäure, Desoxycholsäure und Lithocholsäure, insbesondere Cholesterol.

12. Zusammensetzung nach Anspruch 10, wobei das Molverhältnis der Verbindung zu dem steroidalen Lipid 1:0,5-1,5 beträgt.

13. Zusammensetzung nach Anspruch 3, umfassend die Verbindung und ALC-0315, DSPC und Cholesterol.

14. Verwendung der Verbindung oder des pharmazeutisch unbedenklichen Salzes, des Esters, des Isomers, des Prodrugs und des Solvats davon nach einem der Ansprüche 1 oder 2 oder der Lipidzusammensetzung nach einem der Ansprüche 3 bis 13 bei der Herstellung eines Abgabesystems für eine bioaktive Substanz.

15. Verwendung nach Anspruch 14, wobei die bioaktive Substanz eine niedermolekulare Verbindung, eine Nucleinsäure, ein Peptid oder ein Protein ist.

16. Verwendung nach Anspruch 15, wobei die bioaktive Substanz eine Nucleinsäure ist, wie etwa DNA oder RNA.

17. Verwendung nach Anspruch 15, wobei die RNA ausgewählt ist aus: einer oder mehreren von einer Antisense-RNA, einer saRNA, einer mRNA, einer lncRNA, einer miRNA, einer siRNA, einer piRNA, einer gRNA und einer tsRNA, insbesondere einer mRNA oder einer siRNA.

18. Verwendung nach Anspruch 14 bis 17, wobei es sich bei dem Abgabesystem für die bioaktive Substanz um Lipidnanopartikel (LNPs) handelt.

19. Verwendung der Verbindung oder des pharmazeutisch unbedenklichen Salzes, des Esters, des Isomers, des Prodrugs und des Solvats davon nach einem der Ansprüche 1 oder 2 oder der Lipidzusammensetzung nach einem der Ansprüche 3 bis 13 bei der Herstellung eines Medikaments zum Vorbeugen und/oder Behandeln von Erkrankungen, wobei die Erkrankungen ausgewählt sind aus einem oder mehreren von: Krebs, Entzündung, fibrotischer Erkrankung, Autoimmunerkrankung, durch Infektion mit einem Krankheitserreger verursachter Erkrankung, psychiatrischer Störung, hämatologischer Störung, chromosomaler Erkrankung, genetischer Erkrankung, Bindegewebserkrankung, Verdauungserkrankung, Hals-Nasen-Ohren-Erkrankung, endokriner Erkrankung, Augenerkrankung, reproduktiver Erkrankung, Herzerkrankung, Nierenerkrankung, Lungenerkrankung, Stoffwechselstörung, Munderkrankung, Muskel-Skelett-Erkrankung, Ernährungserkrankung und Hauterkrankung.

20. Verwendung nach Anspruch 19, wobei der Krankheitserreger ein Virus ist, ausgewählt aus: Adenoviridae-, Herpesviridae-, Poxviridae-, Papovaviridae-, Parvoviridae-, Hepadnaviridae-, Polyomaviridae-, Reoviridae-, Picornaviridae-, Caliciviridae-, Togaviridae-, Arenaviridae-, Retroviridae-, Flaviviridae-, Orthomyxoviridae-, Paramyxoviridae-, Bunyaviridae-, Rhabdoviridae-, Filoviridae-, Coronaviridae-, Astroviridae- und Bornaviridae-Viren.

21. Verwendung nach Anspruch 19, wobei das Virus ausgewählt ist aus: HCoV-229E, HCoV-OC43, HCoV-NL63, HCoV-HKU1, SARS-CoV, MERS-CoV, SARS-CoV-2, Influenzavirus, HPIV Typ 1, HPIV Typ 2, HPIV Typ 3, HPIV Typ 4, Sendai-Virus, Mumpsvirus, Masernvirus, respiratorischem Synzytial-Virus, Dengue-Virus, Zika-Virus, Enzephalitis-B-Virus, Chikungunya-Virus, Gelbfiebervirus, Hepatitis-C-Virus, West-Nil-Virus und HPV.

## Revendications

1. Composé comportant la structure suivante : 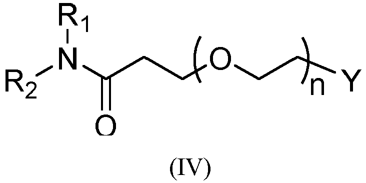 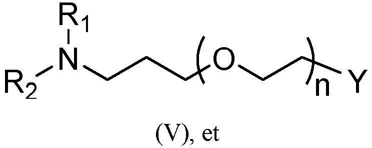 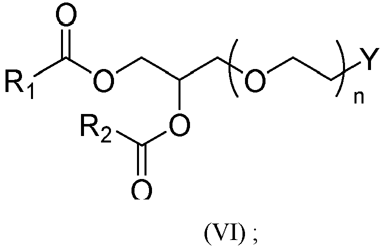
dans laquelle n est un nombre entier de 30 à 90,
R₁ et R₂ sont chacun indépendamment un hydrocarbyle contenant 6 à 30 atomes de carbone, Y est un groupe terminal.

2. Composé selon la revendication 1, ledit composé comportant la structure suivante : 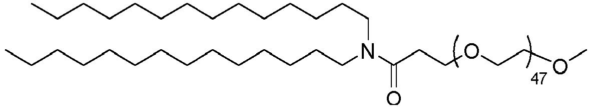 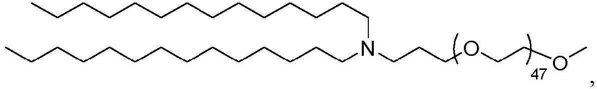 ou

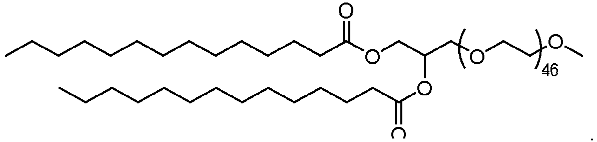

3. Composition lipidique comprenant le composé selon l'une quelconque des revendications 1 ou 2, ou un sel, ester, isomère, promédicament et solvate pharmaceutiquement acceptable de celui-ci.

4. Composition selon la revendication 3, comprenant en outre un lipide cationique.

5. Composition selon la revendication 4, dans laquelle le lipide cationique est choisi parmi : un ou plusieurs de stéaramide (SA), bromure de lauryltriméthylammonium, bromure d'hexadécyltriméthylammonium, bromure de myristyltriméthylammonium, bromure de diméthyldioctadécylammonium (DDAB), 3β-[*N*-(*N'*,*N'*-diméthylaminoéthane)-carbamoyl]cholestérol (cholestérol DC), 1,2-ditétradécanoyl-3-triméthylammonium-propane (DMTAP), 1,2-dioctadécyl-3-triméthylammonium-propane (DOTAP) et les dérivés de DOTAP tels que 1,2-di-(9Z-octadécénoyl)-3-triméthylammonium-propane et 1,2-dihexadécanoyl-3-triméthylammonium-propane, 1,2-di-(9Z-octadécénoyl)-3-diméthylammonium-propane (DODAP) les dérivés de DODAP tels que 1,2-ditétradécanoyl-3-diméthylammonium-propane, 1,2-dihexadécanoyl-3-diméthylammonium-propane et 1,2-dioctadécyl-3-diméthylammoniumpropane, 1,2-di-O-octadécényl-3-triéethylammonium propane (DOTMA), 1,2-dioléoyl-c-(4'-triméthylammonium)-butyryl-sn-glycérol (DOTB), dioctadécylamidoalanyl spermine, SAINT-2, un lipide polycationique 2,3-dioléoyloxy-*N*-[2(spermine-carboxamido)éthyl]-*N*,*N*-diméthyl-1-propanaminium trifluoroacétate (DOSPA) et ((4-hydroxybutyl)azadialkyl)bis(hexane-6,1-diyl)bis(2-hexyldécanoate) (ALC-0315), notamment ALC-0315.

6. Composition selon la revendication 4, dans laquelle le rapport molaire du composé au lipide cationique est de 1:0,01-0,1.

7. Composition selon les revendications 3 à 6, ladite composition comprenant en outre un lipide neutre.

8. Composition selon la revendication 7, dans laquelle le lipide neutre est choisi parmi : un ou plusieurs de 1,2-distéaroyl-sn-glycéro-3-phosphocholine (DSPC), 1,2-dipalmitoyl-sn-glycéro-3-phosphocholine (DPPC), 1,2-dioléoyl-sn-glycéro-3-phosphoéthanolamine (DOPE), 1,2-dipalmitoyl-sn-glycéro-3-phosphoéthanolamine (DPPE), 1,2-dimyristoyl-sn-glycéro-3-phosphoéthanolamine (DMPE), 2-dioléoyl-sn-glycéro-3-phospho-(1'-rac-glycérol) (DOPG), oléoyl phosphatidylcholine (POPC) et 1-palmitoyl-2-oléoyl phosphatidyléthanolamine (POPE), notamment DSPC.

9. Composition selon la revendication 7, dans laquelle le rapport molaire du composé au lipide neutre est de 1:0,1-0,5.

10. Composition selon l'une quelconque des revendications 3 à 9, ladite composition comprenant en outre un lipide stéroïde.

11. Composition selon la revendication 10, dans laquelle le lipide stéroïde est choisi parmi : avenastérol, β-sitostérol, brassicastérol, ergocalciférol, campestérol, cholestanol, cholestérol, coprostérol, déshydrocholestérol, desmostérol, dihydroergocalciférol, dihydrocholestérol, dihydroergostérol, campestérol, épicholestérol, ergostérol, fucostérol, hexahydrostérol, hydroxycholestérol, lanostérol, photostérol, fucostérol, sitostanol, sitostérol, stigmastanol, stigmastérol, acide cholique, acide glycocholique, acide taurocholique, acide désoxycholique et acide lithocholique, notamment cholestérol.

12. Composition selon la revendication 10, dans laquelle le rapport molaire du composé au lipide stéroïde est de 1:0,5-1,5.

13. Composition selon la revendication 3, comprenant le composé, et ALC-0315, DSPC et cholestérol.

14. Utilisation du composé, ou du sel, de l'ester, de l'isomère, du promédicament et du solvate pharmaceutiquement acceptable de celui-ci, selon l'une quelconque des revendications 1 ou 2, ou composition lipidique selon l'une quelconque des revendications 3 à 13, dans la préparation d'un système d'administration d'une substance bioactive.

15. Utilisation selon la revendication 14, dans laquelle la substance bioactive est un composé à petites molécules, un acide nucléique, un peptide ou une protéine.

16. Utilisation selon la revendication 15, dans laquelle la substance bioactive est un acide nucléique, tel que l'ADN ou l'ARN.

17. Utilisation selon la revendication 15, dans laquelle l'ARN est choisi parmi : un ou plusieurs d'un ARN antisense, ARNsa, ARNm, ARNlnc, ARNmi, ARNsi, ARNpi, ARNg et ARNts, notamment un ARNm ou ARNsi.

18. Utilisation selon la revendication 14 ou 17, dans laquelle le système d'administration de la substance bioactive est constitué de nanoparticules lipidiques (LPN).

19. Utilisation du composé, ou du sel, de l'ester, de l'isomère, du promédicament et du solvate pharmaceutiquement acceptable de celui-ci, selon l'une quelconque des revendications 1 ou 2, ou composition lipidique selon l'une quelconque des revendications 3 à 13, dans la préparation d'un médicament destiné à prévenir et/ou traiter des maladies, lesdites maladies étant choisies parmi un ou plusieurs de : cancer, inflammation, maladie fibrotique, maladie autoimmune, maladie causée par une infection par un pathogène, trouble psychiatrique, trouble hématologique, maladie chromosomique, maladie génétique, maladie du tissu conjonctif, maladie digestive, maladie des oreilles, du nez et de la gorge, maladie endocrinienne, maladie oculaire, maladie liée à la reproduction, maladie cardiaque, maladie rénale, maladie pulmonaire, trouble métabolique, maladie buccale, maladie musculosquelettique, maladie nutritionnelle et maladie cutanée.

20. Utilisation selon la revendication 19, dans laquelle le pathogène est un virus choisi parmi : les virus Adenoviridae, Herpesviridae, Poxviridae, Papovaviridae, Parvoviridae, Hepadnaviridae, Polyomaviridae, Reoviridae, Picornaviridae, Caliciviridae, Togaviridae, Arénaviridae, Retroviridae, Flaviviridae, Orthomyxoviridae, Paramyxoviridae, Bunyaviridae, Rhabdoviridae, Filoviridae, Coronaviridae, Astroviridae et Bornaviridae.

21. Utilisation selon la revendication 19, dans laquelle le virus est choisi parmi : HCoV-229E, HCoV-OC43, HCoV-NL63, HCoV-HKU1, SARS-CoV, MERS-CoV, SARS-CoV-2, virus de la grippe, HPIV de type 1, HPIV de type 2, HPIV de type 3, HPIV de type 4, virus Sendai, virus des oreillons, virus de la rougeole, virus respiratoire syncytial, virus de la Dengue, virus Zika, virus de l'encéphalite B, virus du Chikungunya, virus de la fièvre jaune, virus de l'hépatite C, virus du Nil Occidental et HPV.
